# FASCICULE DE BREVET EUROPEEN

(11) **EP 0 471 599 B1**
(45) Date de publication et mention de la délivrance du brevet: **17.11.1994**
(21) Numéro de dépôt: 91402090.4
(22) Date de dépôt: 25.07.1991
(51) Int. Cl.: C07C 5/41, C07C 2/00

(54) **Procédé d'aromatisation des hydrocarbures contenant de 5 à 9 atomes de carbone par molécule**
Verfahren zur Aromatisierung von Kohlenwasserstoffen mit 5 bis 9 Kohlenstoffatomen in dem Molekül
Process for the aromatisation of hydrocarbons having 5 to 9 carbon atoms in the molecule

(30) Priorité: 16.08.1990 FR 9010451
(43) Date de publication de la demande: 19.02.1992
(73) Titulaire: INSTITUT FRANCAIS DU PETROLE, 92502 Rueil-Malmaison (FR)
(72) Inventeur: Bournonville, Jean-Paul, F-95000 Cergy Pontoise (FR); Raatz, Francis, F-57500 Saint-Avold (FR); Juguin, Bernard, Décédé (FR)

(56) Documents cités:
- EP-A- 0 351 311
- WO-A-89/04818
- FR-A- 2 144 702
- US-A- 4 435 283

## Description

La présente invention concerne un procédé d'aromatisation des hydrocarbures comprenant entre 5 et 9 atomes de carbone par molécule en présence d'un catalyseur composite comprenant d'une part une zéolithe de structure MFI contenant du silicium, de l'aluminium et d'autre part un métal de la famille du platine déposé sur un support oxyde réfractaire, sur lequel sont ajoutés au moins un additif métallique choisi dans le groupe constitué par l'étain, le germanium, l'indium et le plomb et au moins un métal alcalin ou un métal alcalino- terreux.

Les catalyseurs à base de zéolithes dopés au gallium, zinc, platine, sont connus pour être actifs et sélectifs en aromatisation du propane et du butane. Classiquement les hydrocarbures à plus de 6 atomes de carbone par molécule sont transformés en aromatiques par réformage catalytique en utilisant des catalyseurs du type alumine acide dopée au platine, métal auquel on peut ajouter de l'étain, du rhénium etc. Ces catalyseurs de réformage sont cependant très peu performants pour l'aromatisation des hydrocarbures comprenant 5 atomes de carbone par molécule, et dans une moindre mesure pour ceux comprenant 6 atomes de carbone par molécule. Il y a donc un grand intérêt pratique à trouver des catalyseurs performants pour l'aromatisation de coupes riches en hydrocarbures du type C₅-C₆.

La réaction d'aromatisation des hydrocarbures comprenant plus de 5 atomes de carbone par molécule en présence de catalyseurs zéolithiques a déjà fait l'objet de plusieurs brevets et publications. Plusieurs systèmes catalytiques à base de zéolithe MFI sont revendiqués, ces systèmes pouvant être distingués par les ajouts qu'ils contiennent. Schématiquement on peut distinguer :
i) les systèmes contenant du gallium (G. Berti, J. Moore, L. Salusinszki, D. Seddon, Aust. J. Chem., 42, p. 2095, 1989).
ii) les systèmes contenant du zinc (O. Anunziata, O. Orio, L. Pierella, M. Aguirre, React. Kin. Catal. Lett., 39(1), 75, 1989 ; J. Kanai, N. Kawata, J. Catal., 114, 284, 1988).

Ces systèmes souffrent tous d'un défaut important, à savoir une sélectivité élevée en méthane. Pour améliorer les performances de ces systèmes catalytiques plusieurs solutions ont été proposées dont l'ajout de platine (Z. Jin, Y. Makino, A. Miyamoto, T. Inui, Chem. Express, 2, p. 515, 1987). L'utilisation d'une zéolithe MFI non acide dopée avec divers éléments métalliques a également été revendiquée (Y. Chen, et al. WO 8904818).

On a découvert que des performances améliorées par rapport à ce qui est connu dans l'art antérieur peuvent être obtenues en aromatisation des hydrocarbures contenant entre 5 et 9 atomes de carbone par molécule en utilisant un catalyseur composite particulier.

Ce catalyseur comprend une zéolithe de structure MFI d'une part, et d'autre part un support ou une matrice généralement amorphe sur lequel est déposé un métal noble de la famille du platine et au moins un métal additionnel choisi dans le groupe constitué par l'étain, le germanium, le plomb, et l'indium, ledit support contenant également au moins un métal alcalin ou au moins un métal alcalinoterreux choisi notamment dans le groupe constitué par le lithium, le sodium, le potassium, le rubidium, le césium, le baryum, le calcium, le béryllium, le magnésium et le strontium.

La zéolithe de structure MFI constituant une partie du catalyseur de la présente invention, peut être préparée par toutes les techniques connues dans l'art antérieur. La synthèse de ladite zéolithe MFI peut être réalisée en milieu classique OH⁻ , en présence ou non de structurants organiques et/ou d'alcool. La synthèse de la zéolithe MFI en milieu OH⁻ selon les techniques connues dans l'art antérieur est largement décrite dans le document suivant : Synthesis of High Silica Zeolites, P. Jacobs and J. Martens, Studies in Surface Science and Catalysis, Volume 33, Elsevier editor, 1987. La zéolithe MFI peut également être synthétisée dans des milieux moins classiques comme par exemple le milieu fluorure (Brevet Européen EP-A-172068 de C.F.R).

Après synthèse, la zéolithe MFI est transformée en une forme hydrogène par élimination totale ou partielle des composés organiques et/ou des cations alcalins ou alcalino-terreux qu'elle contient éventuellement après la synthèse. Toutes les techniques connues dans l'art antérieur peuvent être utilisées pour passer à la forme hydrogène, comme par exemple les calcinations sous atmosphère oxydante ou non, les échanges ioniques suivis ou non de calcination, les traitements chimiques divers etc.

Toutes les zéolithes MFI synthétisées dans le système Si-Al conviennent pour la présente invention. Cependant leur rapport Si/Al sera généralement supérieur à 7, de préférence supérieur à 25 et de préférence compris entre 40 et 1 000.

Les supports des métaux ajoutés à la zéolithe sont généralement choisis parmi les oxydes des métaux des groupes II, III et/ou IV de la classification périodique des éléments, tels que par exemple, les oxydes de magnésium, d'aluminium, de titane, de zirconium, de thorium ou de silicium, pris seuls ou en mélange entre eux ou avec des oxydes d'autres éléments de la classification périodique, tels que par exemple le bore. On peut aussi utiliser du charbon.

Le support préféré est l'alumine ; la surface spécifique de l'alumine peut avantageusement être comprise entre 50 et 600 m² par gramme, de préférence entre 150 et 400 m²/g.

Le catalyseur composite de la présente invention peut être préparé suivant deux voies dont le principe est donné ci-après.
Première voie : mélange de la zéolithe MFI avec le support ; ce mélange peut être réalisé entre deux poudres, entre les deux solides mis en forme, entre une poudre et un des solides mis en forme. On peut également mettre en forme conjointement les deux solides par toutes les techniques connues dans l'art antérieur : pastillage, extrusion, dragéification, coagulation en goutte, séchage par atomisation. Lors de ces opérations de mise en forme, on pourra si nécessaire ajouter un additif de mise en forme (silice etc). Après mélange et/ou mise en forme, on procède au dépôt des divers agents actifs sur le support (en présence donc de la zéolithe).
Deuxième voie : on dépose préalablement les agents actifs sur le support, que l'on mélange ou que l'on met en forme avec la zéolithe MFI dans les mêmes conditions que précédemment. Dans une variante, la zéolithe pourra être introduite dans le catalyseur composite à l'une quelconque des étapes de dépôt des agents actifs sur le support.

La méthode préférée de préparation consiste à déposer les agents actifs sur le support puis à introduire la zéolithe dans le catalyseur final par mise en forme des deux poudres. La mise en forme sera de préférence réalisée après un broyage micronique, qui pourra être réalisé en utilisant la technique de broyage humide.

Le catalyseur composite contient entre 1 et 99 % en poids de zéolithe, le complément à 100 % étant constitué par le support chargé en différents agents actifs. La proportion respective de zéolithe et de support varie dans une large gamme, car elle dépend d'une part' du rapport Si/Al de la zéolithe et d'autre part de la teneur en agents actifs du support.

La partie du catalyseur composite comprenant du métal noble est généralement préparée selon des méthodes classiques consitant à imprégner le support au moyen de solutions de composés des métaux que l'on désire introduire. On utilise soit une solution commune de ces métaux, soit des solutions distinctes pour le métal de la famille du platine et pour le ou les métaux additionnels. Quand on utilise plusieurs solutions, on peut procéder à des séchages et/ou calcinations intermédiaires. On termine habituellement par une calcination par exemple entre environ 500 et 1000°C, de préférence en présence d'oxygène libre, par exemple en effectuant un balayage d'air.

Le platine (ou éventuellement un autre métal noble du groupe du platine) peut être incorporé dans le support par imprégnation de ce support à l'aide d'une solution adéquate aqueuse ou non renfermant un sel ou un composé du métal noble. Le platine est généralement introduit dans le support sous forme d'acide chloroplatinique mais peuvent également être utilisés des composés tels que le chloroplatinate d'ammonium, le dichlorure de platine dicarbonyle, l'acide hexahydroxyplatinique, le chlorure de palladium, le nitrate de palladium.

L'élément choisi dans le groupe constitué de l'étain, du germanium, du plomb et de l'indium peut être introduit par l'intermédiaire de composés tels que par exemple les chlorures, les bromures et le nitrate d'étain, les halogénures, nitrate, acétate et carbonate de plomb, le chlorure et l'oxalate de germanium, le nitrate et le chlorure d'indium.

L'élément choisi dans le groupe constitué par les alcalins et alcalino-terreux peut être introduit par l'intermédiaire de composés tels que les halogénures, les nitrates, les carbonates, cyanures, oxalates.

Un procédé de fabrication comprend par exemple les étapes suivantes :
a) Introduction sur le support d'au moins un élément choisi dans le groupe constitué par les alcalins et les alcalino-terreux.
b) Calcination du produit obtenu à l'étape a).
c) Introduction sur le support d'au moins un métal noble de la famille du platine.
d) Calcination du produit obtenu à l'étape c).
e) Introduction sur le produit obtenu à l'étape b) d'au moins un métal additionnel M.

Parmi les composés du ou des métaux de la famille du platine que l'on emploie dans la présente invention, on peut citer à titre d'exemple les complexes ammoniés.

On citera en particulier dans le cas du platine les sels de platine IV hexamines de formule (Pt(NH₃)₆X₄ où X est un atome d'halogène choisi dans le groupe formé par le fluor, le brome et l'iode et de préférence X est un atome de chlore ; les sels de platine IV halogénopentammines de formule (Pt X(NH₃)₅)X₃ ; les platine IV tétrahalogénodiammines de formule Pt X₄(NH₃)₂ où X a la signification donnée ci-dessus ; les complexes du platine avec les halogènespolycétones et les composés halogénés polycétoniques de formule H(Pt(aca)₂X) dans lesquels X a la signification donnée ci-dessus et aca représente le reste de formule C₅H₇O₂ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables on peut citer les hydrocarbures paraffiniques, naphténiques, ou aromatiques et les composés organiques halogénés ayant par exemple de 1 à 12 atomes de carbone dans leur molécule. On citera en particulier le n-heptane, le méthylcyclohexane, le toluène et le chloroforme. On peut aussi utiliser des mélanges de solvants.

Les supports sont les supports classiques tels que ceux définis ci-dessus et contenant déjà par exemple un alcalin ou un alcalino-terreux.

Après introduction du métal noble de la famille du platine, le produit obtenu est éventuellement séché puis calciné de préférence à une température d'environ 400 à 1000°C.

Après cette calcination on procède à l'introduction du ou des métaux additionnels ; éventuellement avant d'introduire ledit métal M on procède à une réduction à l'hydrogène à haute température, par exemple de 300 à 500°C. Cette réduction peut consister par exemple en une montée lente de la température sous courant d'hydrogène jusqu'à la température maximale de réduction, comprise par exemple entre 300 et 500°C et de préférence entre 350 et 450°C suivie d'un maintien sous hydrogène pendant 1 à 6 heures à cette température.

Le métal additionnel M peut être introduit avant ou après l'introduction du métal noble. S'il est introduit avant le métal noble, le composé utilisé sera choisi dans le groupe constitué par les halogénures, nitrates, acétates, carbonates, oxalates du métal additionnel. L'introduction sera avantageusement effectuée en solution aqueuse. Dans ce cas, avant de procéder à l'introduction du métal noble, on procède à une calcination sous air à une température comprise entre 400 et 1000° C.

Le métal additionnel M peut être introduit après l'introduction du métal noble sous la forme d'au moins un compose organique choisi dans le groupe formé par les complexes, en particulier les complexes polycétoniques, des métaux M et les hydrocarbylmétaux tels que les alkyles, les cycloalkyles, les aryles, les alkyaryles et les arylalkyles métaux.

L'introduction du métal M est avantageusement effectuée à l'aide d'une solution dans un solvant organique du composé organométallique dudit métal M. On peut également employer des composés organohalogénés des métaux M. Comme composés de métaux M on citera en particulier le tétrabutyl-étain, le tétraméthyl-étain, le tétrapropyl-germanium, le tétraéthyl-plomb, l'acétylacétonate d'indium, le triphénylindium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer le n-heptane, le méthylcyclohexane, le toluène et le chloroforme. On peut citer des mélanges de solvants définis ci-dessus.

Cette méthode d'introduction du métal M a déjà été décrite dans le brevet US-A-4548918. Mais la combinaison de la méthode d'introduction du métal de la famille du platine et de la méthode d'introduction du métal M engendre une synergie particulière.

La partie du catalyseur composite, contenant le métal noble renferme en poids par rapport au support (a) environ 0,01 à 2 % et plus particulièrement environ 0,1 à 0,5 % d'au moins un métal noble de la famille du platine, (b) environ 0,005 à 0,60 %, et de préférence 0,01 à 0,50 d'étain ou 0,005 à 0,70 % de préférence environ 0,01 à 0,6 % et plus particulièrement 0,02 à 0,50 % d'au moins un métal choisi dans le groupe constitué par le germanium, le plomb et l'indium, (c) environ 0,01 à 2 % et plus particulièrement environ 0,1 à 0,6 % d'au moins un métal choisi dans le groupe constitué par les alcalins et les alcalino-terreux et de préférence le lithium et le potassium et leurs mélanges.

Lorsqu'il y a au moins deux métaux de la famille étain, germanium, plomb et indium, la teneur globale en métaux de cette famille est d'environ 0,02 à 1,20 % et de préférence 0,02 à 1,0 % et plus particulièrement 0,03 à 0,80 %.

Dans le procédé de l'invention, à l'issue de la préparation de la partie du catalyseur composite contenant le métal noble, celui-ci est généralement calciné entre 450 et 1000°C mais le catalyseur, à l'issue de la calcination peut subir avantageusement un traitement d'activation sous hydrogène à haute température, par exemple 300-500°C, afin d'obtenir une phase métallique plus active. La procédure de ce traitement sous hydrogène consiste par exemple en une montée lente de la température sous courant d'hydrogène jusqu'à la température maximale de réduction, comprise par exemple entre 300 et 500°C et de préférence entre 350 et 450°C, suivie d'un maintien pendant 1 à 6 heures à cette température.

Ce type de préparation du catalyseur conduit à un solide dans lequel les métaux sont répartis de façon homogène dans tout le volume du grain de catalyseur, et sont dans un état métallique après le traitement de réduction sous balayage d'hydrogène entre 300 et 500°C et maintien pendant 1 à 6 heures sous hydrogène à la température finale choisie.

Les exemples suivants illustrent l'invention sans en limiter la portée.

### EXEMPLE 1

On se propose de transformer une charge consituée d'un mélange d'hydrocarbures contenant 5 ou 6 atomes de carbone en présence d'un catalyseur à base de mélange d'une zéolithe MFI de rapport Si/Al = 75 et d'une alumine contenant du platine, de l'étain et du lithium (l'alumine renfermant le platine, l'étain et du lithium est appelée ci-après catalyseur A).
. On utilise une zéolithe MFI forme H fournie par la société CONTEKA sous la référence CBV 1502. Cette zéolithe MFI est caractérisée par un rapport Si/Al égal à 75, une teneur en sodium égale à 0,016 % pds et un volume poreux mesuré par adsorption d'azote à 77 K de 0,174 cm³ g⁻¹.
. Préparation de l'alumine renfermant les métaux choisis (catalyseur A).

L'alumine renfermera 0,30 % de platine en poids, 0,3 % d'étain et 0,5 % de lithium.

L'alumine utilisée a une surface spécifique de 240 m²/g et un volume poreux de 0,58 cm³/g.

On ajoute à 100 grammes de support d'alumine, 100 cm³ d'une solution aqueuse de nitrate de lithium.

On laisse en contact 6 heures, on essore, on sèche 1 heure à 100-120°C puis on calcine 2 heures à 530°C.

Sur le produit calciné contenant le lithium on procède à l'imprégnation de l'étain : une solution aqueuse d'acétate d'étain est mise en contact avec le support d'alumine à raison de 100 cm³ de solution pour 100 grammes de support pendant 6 heures. Le solide obtenu est alors essoré et séché 1 heure à 100-120°C puis calciné à 530°C.

On procède alors sur le solide calciné contenant le lithium et l'étain à l'imprégnation du platine, en ajoutant au solide 100 cm³ d'une solution d'acétylacétonate de platine dans le toluène. La concentration en platine de cette solution est égal à 3 grammes par litre. On laisse 6 heures en contact, on sèche 1 heure à 100-120°C puis on calcine 2 heures à 530°C. Puis on réduit sous courant d'hydrogène sec pendant 2 heures à 450°C.

### EXEMPLE 2

Ces produits ont été testés en transformation d'une charge C₅-C₆ dont la composition est la suivante (exprimée en % pds) :

| | | |
|---|---|---|
| | C₅ | 90,0 % |
| Paraffines | C₆ | 5,4 % |
| | C₅ | 3,7 % |
| Naphtènes | C₆ | 0,9 % |

Les conditions opératoires sont les suivantes :

| | |
|---|---|
| - Température | 480°C |
| - Pression | 2,5 bars |
| - pph | 3 h⁻¹ |

Il a, tout d'abord été procédé à un test des deux solides seuls : zéolithe HMFI et catalyseur A, puis mélange des deux.

Les résultats du test sont reportés dans le tableau I.

**TABLEAU I**

| Catalyseur | Conversion (% poids) | Sélectivité (% poids) | | | | | |
|---|---|---|---|---|---|---|---|
| | | CH₄ | C₂H₆ + C₂H₄ | C₃H₈ + C₃H₆ | C₄H₁₀ | Aromatiques | C₅+C₆ oléfines |
| Zéolithe MFI (comparatif) | 92 | 30 | 25 | 15 | 20 | 10 | - |
| Catalyseur A (Pt+Sn+Li/Alumine) | 50 | 2 | 2 | 5 | 1 | - | 90 |
| Mélange (50 % zéolithe) (50 % catalyseur A) | 90 | 6 | 12 | 14 | 8 | 60 | - |

### EXEMPLE 3

Dans cet exemple, on utilise une zéolithe MFI de rapport Si/Al = 75 synthétisée en absence de composé organique d'après un protocole de synthèse décrit dans "Studies in Surface Science and Catalysis", vol. 33, 1987, p. 134. Après synthèse, la zéolithe subit les traitements suivants :
- trois échanges dans NH₄NO₃ 10N à 100°C pendant 6 heures
- calcination sous air à 550°C 4 heures avec un débit de 3 l/h/g
- 50 % (poids) de zéolithe MFI de rapport Si/Al = 75, ont été mélangés à 50 % (poids) des catalyseurs A, B, C, D respectivement.

Les résultats obtenus dans les conditions opératoires précédentes (exemple 2) sont rassemblés dans le tableau II présenté ci-dessous.

Les catalyseurs B, C, D sont préparés de la même manière que le catalyseur A sauf que l'étain est remplacé respectivement par le germanium, le plomb et l'indium. Les précurseurs utilisés sont respectivement l'oxalate de germanium, l'acétate de plomb et le nitrate d'indium.

**TABLEAU II**

| Catalyseur | Conversion (% poids) | Sélectivité (% poids) | | | | | |
|---|---|---|---|---|---|---|---|
| | | CH₄ | C₂H₆ + C₂H₄ | C₃H₈ + C₃H₆ | C₄H₁₀ | Aromatiques | Oléfines C₅ + C₆ |
| Zéolithe MFI + Catalyseur A (Pt+Sn+Li) | 90 | 6 | 12 | 14 | 8 | 60 | - |
| Zéolithe MFI + Catalyseur B (Pt+Ge+Li) | 89 | 7 | 11 | 15 | 7 | 60 | - |
| Zéolithe MFI + Catalyseur C (Pt+Pb+Li ) | 92 | 5 | 12 | 14 | 10 | 58 | - |
| Zéolithe MFI + Catalyseur D (Pt+In+Li) | 91 | 5 | 12 | 15 | 9 | 59 | - |

### EXEMPLE 4

On se propose de transformer une charge de composition identique à celle décrite dans l'exemple 1 en présence d'un catalyseur à base d'un mélange de zéolithe MFI de rapport Si/Al = 75, décrite dans l'exemple 1, et d'une alumine contenant du platine, de l'étain et du lithium et appelée ci-après catalyseur E.

Préparation de l'alumine renfermant les métaux choisis (catalyseur E) :
L'alumine renfermera 0,3 % de platine, en poids, 0,3 % d'étain et 0,5 % de lithium.

On ajoute à 100 grammes de support d'alumine, 100 cm3 d'une solution aqueuse de nitrate de lithium, on laisse en contact 6 heures, on essore, on sèche en 1 heure à 100-120°C puis on calcine sous courant d'air sec 2 heures à 530°C.

Sur le produit calciné, contenant le lithium, on procède à l'imprégnation du platine de la même façon que le produit (A).

Après réduction, le produit contenant le lithium et le platine est immergé dans le n-heptane à raison de 100 grammes de solide pour 300 cm³ de solvant hydrocarboné. Ensuite on injecte, dans le n-heptane contenant le catalyseur, 3 grammes d'une solution de tétra-n-butyl étain dans le n-heptane (à 10 % en étain). Le contact entre le solide contenant le platine et la solution de tétra-n butyl étain est maintenu pendant 6 heures à la température de reflux de l'heptane. La solution d'imprégnation est alors évacuée et l'on procède à 3 lavages par du n-heptane pur à la température de reflux du n-heptane. Le catalyseur est ensuite séché. Il peut alors subir soit une calcination sous air pendant 2 heures à 500°C suivie d'une réduction sous courant d'hydrogène à 450°C avant d'être chargé dans le réacteur, soit subir après séchage une réduction directe sous courant d'hydrogène à 450°C pendant 2 heures avant d'être chargé dans le réacteur.

Le tableau III donne les résultats comparatifs des mélanges zéolithe MFI et catalyseurs A et E respectivement.

**TABLEAU III**

| Catalyseur | Conversion (% poids) | Sélectivité (% poids) | | | | | |
|---|---|---|---|---|---|---|---|
| | | CH₄ | C₂H₆ + C₂H₄ | C₃H₈ + C₃H₆ | C₄H₁₀ | Aromatiques | Oléfines C₅ et C₆ |
| Zéolithe MFI + Catalyseur A | 90 | 6 | 12 | 14 | 8 | 60 | - |
| Zéolithe MFI + Catalyseur E | 92 | 5 | 11 | 15 | 7 | 62 | - |

## Revendications

1. Procédé d'aromatisation d'hydrocarbures contenant 5 à 9 atomes de carbone par molécule en présence d'un catalyseur renfermant (a) une zéolithe MFI, (b) une matrice renfermant au moins un métal noble de la famille du platine, au moins un métal additionnel choisi dans le groupe constitué par l'étain, le germanium, le plomb et l'indium et au moins un métal alcalin ou alcalino-terreux, caractérisé en ce que ledit catalyseur renferme en poids :
(a) 1 à 99% de zéolithe MFI
(b) 99 à 1 % d'une matrice autre que ladite zéolithe MFI renfermant 0,01 à 2 % d'un métal de la famille du platine, 0,005 à 0,60 % de métal additionnel lorsque celui-ci est l'étain ou 0,005 à 0,70 % de métal additionnel lorsque celui-ci est le germanium, le plomb ou l'indium, et 0,01 à 2 % d'au moins un métal alcalin ou alcalino-terreux.

2. Procédé selon la revendication 1 dans lequel la matrice du catalyseur est l'alumine.

3. Procédé selon l'une des revendications 1 et 2 dans lequel la matrice du catalyseur contient en poids 0,1 à 0,5 % d'au moins un métal noble de la famille du platine, 0,01 à 0,5 % d'étain ou 0,01 à 0,6 % de germanium, d'étain ou de plomb et 0,1 à 0,6 % d'au moins un métal alcalin ou alcalino-terreux.

4. Procédé selon l'une des revendications 1 à 3 dans lequel le catalyseur renferme à titre desdits métaux additionnels de l'étain et un métal choisi dans le groupe constitué par l'étain, le germanium et le plomb, la teneur totale en métaux additionnels étant comprise entre 0,02 et 1,20 %.

5. Procédé selon l'une des revendications 1 à 4 dans lequel le catalyseur renferme une matrice d'alumine, du platine, au moins un métal additionnel dont l'étain et du lithium ou du potassium.

## Claims

1. A process for the aromatisation of hydrocarbons containing from 5 to 9 carbon atoms per molecule in the presence of a catalyst containing (a) a zeolite MFI, and (b) a matrix containing at least one noble metal of the platinum family, at least one additional metal selected from the group formed by tin, germanium, lead and indium, and at least one alkali or alkaline-earth metal, characterised in that said catalyst contains by weight:
(a) from 1 to 99% of zeolite MFI, and
(b) fro 99 to 1% of a matrix other than said zeolite MFI containing from 0.01 to 2% of a metal of the platinum family, from 0.005 to 0.60% of additional metal when same is tin or from 0.005 to 0.70% of additional metal when same is germanium, lead or indium, and from 0.01 to 2% of at least one alkali or alkaline-earth metal.

2. A process according to claim 1 wherein the matrix of the catalyst is alumina.

3. A process according to one of claims 1 and 2 wherein the matrix of the catalyst contains by weight from 0.1 to 0.5% of at least one noble metal of the platinum family, from 0.01 to 0.5% of tin or from 0.01 to 0.6% of germanium, tin or lead and 0.1 to 0.6% of at least one alkali or alkaline-earth metal.

4. A process according to one of claims 1 to 3 wherein the catalyst contains as said additional metals tin and a metal selected from the group formed by tin, germanium and lead, the total amount of additional metals being between 0.02 and 1.20%.

5. A process according to one of claims 1 to 4 wherein the catalyst contains a matrix of alumina, platinum, and at least one additional metal including tin and lithium or potassium.

## Patentansprüche

1. Verfahren zur Aromatisierung von Kohlenwasserstoffen, welche 5 bis 9 Kohlenstoffatome pro Molekül umfassen, in Gegenwart eines Katalysators, der
(a) einen MFI-Zeolith und
(b) eine Matrix umfaßt, die wenigstens ein Edelmetall der Platinfamilie, wenigstens ein zusätzliches Metall, das aus der Gruppe, die aus Zinn, Germanium, Blei und Indium besteht,ausgewählt ist und wenigstens ein Alkali- oder Erdalkalimetall umfaßt, dadurch gekennzeichnet, daß der Katalysator, bezogen auf das Gewicht, umfaßt:
(a) 1 bis 99% MFI-Zeolith
(b) 99 bis 1% einer vom MFI-Zeolith verschiedenen Matrix, welche 0.01 bis 2% eines Metalles der Platinfamilie, 0.005 bis 0.60 eines zusätzlichen Metalles , wenn dieses Zinn ist, oder 0.005 bis 0.70 eines zusätzlichen Metalles, wenn dieses Germanium, Blei oder Indium ist, und 0.01 bis 2% wenigstens eines Alkali- oder Erdalkalimetalles umfaßt.

2. Verfahren nach Anspruch 1, worin die Katalysatormatrix Aluminiumoxid ist.

3. Verfahren nach einem der Ansprüche 1 und 2, worin die Katalysatormatrix, bezogen auf das Gewicht, 0.1 bis 0.5% wenigstens eines Edelmetalles der Platinfamilie, 0.01 bis 0.5% Zinn oder 0.01 bis 0.6% Germanium. Zinn oder Blei und 0.1 bis 0.6% wenigstens eines Alkali- oder Erdalkalimetalles enthält.

4. Verfahren nach einem der Ansprüche 1 bis 3, worin der Katalysator, bezogen auf die zusätzlichen Metalle, Zinn und ein Metall, das aus der Gruppe, die aus Zinn, Germanium oder Blei besteht, ausgewählt ist umfaßt, wobei das Gesamtgewicht der zusätzlichen Metalle zwischen 0.02 und 1.20% liegt.

5. Verfahren nach einem der Ansprüche 1 bis 4, worin der Katalysator eine Aluminiumoxid-Matrix, Platin und wenigstens ein zusätzliches Metall wie Zinn, Lithium oder Kalium umfaßt.
